# EUROPEAN PATENT APPLICATION

(11) **EP 1 642 592 A1**
(43) Date of publication of application: **05.04.2006**
(21) Application number: 04747329.3
(22) Date of filing: 02.07.2004
(51) Int. Cl.: A61K 45/00, A61K 31/7056, A61P 35/00

(54) **REMEDY FOR SARCOIDOSIS AND METHOD OF TREATING THE SAME**

(30) Priority: 03.07.2003 JP 2003270809
(71) Applicant: Japan Science and Technology Corporation, Kawaguchi-shi, Saitama 332-0012 (JP)
(72) Inventor: MATSUSHIMA, Kouji, Matsudo-shi, Chiba 2710092 (JP); NISHIWAKI, Tetsu, Tokyo 1080023 (JP); YONEYAMA, Hiroyuki, Bunkyo-ku, Tokyo 1120002 (JP)
(74) Representative: Harding, Charles Thomas
(86) International application number: PCT/JP2004/009861
(87) International publication number: WO 2005/002623

(57) **Abstract**

The present invention provides a remedy for sarcoidosis, one of systemic granulomatous diseases, and a method for treating sarcoidosis. A remedy for sarcoidosis containing a Propioni bacterium acnes-targeting antibiotic such as minocycline hydrochloride and clindamycin as an active component is prepared. Further, sarcoidosis is treated by administering this remedy for sarcoidosis to sarcoidosis patients.

## Description

### Technical Field

The present invention relates to a remedy for sarcoidosis containing a Propionibacterium acnes-targeting antibiotic as an active component, and a method for treating sarcoidosis wherein the remedy is administered to sarcoidosis patients.

### Background Art

Sarcoidosis is one of the best-known systemic granulomatous diseases, and despite a number of intensive investigations, its etiology has remained unknown for more than 100 years (for example, see N. Engl. J. Med. 336, 1224-1234, 1997). The lung is the organ most commonly affected, and untreated pulmonary granulomatous inflammation results in impedance of gaseous exchange, and often leads to irreversible fibrotic changes and a poor prognosis. The incidence of long-term respiratory problems with sustained pulmonary inflammation or fibrosis in the general population is quite high. As the lung is constantly confronted with airborne substances, including pathogens, many researchers have directed their attention to identification of potential causative transmissible agents and their contribution to the mechanism of pulmonary granuloma formation (for example, see Clin. Exp. Allergy. 31, 543-554, 2001; Curr. Opin. Pulm. Med. 8, 435-440, 2002).

Due to their clinical and immunopathological similarities , it is considered that the most common mycobacterial infection, tuberculosis, may be related to sarcoidosis. However, despite use of bacterial culture systems, histological methods, and polymerase chain reaction (PCR), the association between Mycobacterium tuberculosis and sarcoidosis is still under discussion (for example, see non-patent documents Am. J. Respir. Crit. Care Med. 156, 1000-1003, 1997; Hum. Pathol. 28, 796-800, 1997; Thorax. 51, 530-533, 1996). Propionibacterium acnes (P.acnes) is an anaerobic non-spore-forming gram-positive rod that exists indigenously on the skin or the mucosal surface (for example, see Manual of Clinical Microbiology, 587-602, 1995), and has been recently suggested as a major candidate for the causative antigen of sarcoidosis (for example, see Lancet. 361, 1111-1118, 2003). Some studies using quantitative PCR demonstrated that the level of P. acnes genomes in mediastinal or superficial lymph nodes (LNs) of sarcoidosis patients is markedlyhigher than that of controls, suggesting the possibility of "an intrinsic infection" in patients due to P. acnes (for example, see Lancet. 354, 120-123, 1999; J. Clin. Microbiaol. 40, 198-204, 2002; J. Pathol. 198, 541-547, 2002).

The process of inducing pulmonary granuloma formation is considered to comprise the steps of: airborne or blood-borne antigens anchor in the lung; and then antigen-presenting cells (APCs), such as macrophages or dendritic cells (DCs) (for example, see Am. J. Respir. Cell Mol . Biol. 26, 671-679 , 2002), accumulate and surround the antigens for phagocytosis and subsequent antigen presentation (for example, see The Lung. Vol . 1. 2395-2409, 1997). Based on this consideration, methods using antigen-embolization to hold antigens in the lung have been proposed in some animal models of pulmonary granuloma, particularly models for murine schistosomiasis (for example, seeAm. J. Pathol. 158, 1503-1515, 2001; J. Immunol. 166, 3423-3439, 2001). However, long-term antigen deposition at the pulmonary interstitium is not suitable for clinical pulmonary studies, and it is unlikely that disseminated blood-borne antigens are responsible for all cases of pulmonary granuloma.

The object of the present invention is to provide a remedy for sarcoidosis, one of systemic granulomatous diseases, and a method for treating sarcoidosis.

The present inventors have conducted an intensive study to attain the above-mentioned object and obtained the following findings (1) to (7), and the present invention has been completed.
(1) It was found that there is P. acnes phagocytized mainly by macrophages in the lower airway of normal lung, which is believed to be germ-free, by immunostaining of healthy mouse lung with the use of anti-P. acnes monoclonal antibodies.
(2) P. acnes was detected in the lower airway of healthy lung, by RT-PCR using a primer of the 16s ribosomal RNA of P. acnes, supporting (1).
(3) P. acnes was also detected in regional lymph nodes of the normal lung as in (2). Further, P. acnes-specific immune response was observed in a lymphocyte proliferation assay.
(4) Pulmonary/hepatic granulomas were formed by the experiment of intravenous injection of P. acnes-sensitized CD4⁺ T cells into normal mice.
(5) When extrapulmonary (footpads) repeated immunization (400 pg/2-week interval) of mice with P. acnes as an application model of the above-mentioned (4), type Thl granulomas, preferably distributed into the subpleura/bronchovascular bundle, were formed. In these mice, serum calcium/ACE level increased antigen-dose-dependently, and CD4/8 ratio in BAL (bronchoalveolar lavage) lymphocytes was positively correlated with serum calcium level. Further, as an extrapulmonary lesion, abnormal accumulation of hepatic granulomas/CD4⁺ T cells in the red pulp of the spleen was observed. These results closely resemble immunohistological features of pulmonary sarcoidosis.
(6) In order to indicate that P. acnes, which exists indigenously in normal lung, plays an important role in the formation of the above-mentioned sarcoidosis-like pulmonary granulomas, live cells of P. acnes were preadministered one week before the initiation of the repeated immunization, and it was examined whether granuloma formation is enhanced. As a result, granuloma formation was enhanced cell-dose-dependently, and the number of BAL cells was also increased.
(7) With the same objective as that of the above-mentioned (6), the effect of antibacterial operation to P. acnes on the pulmonary granuloma formation was examined. In groups administered with minocycline hydrochloride or clindamycin, whose antibacterial effect on P. acnes has been known, the total number of BAL cells and the number of CD4⁺ cells were decreased, and also, granuloma formation was suppressed in comparison to groups administered with PBS.

### Disclosure of the Invention

The present invention relates to: (1) a remedy for sarcoidosis containing a Propionibacterium acnes-targeting antibiotic as an active component; (2) the remedy for sarcoidosis according to (1), wherein the Propionibacterium acnes-targeting antibiotic is one or more antibiotics selected from penicillin antibiotics,cephalosporin antibiotics,macrolide antibiotics, lincomycin antibiotics, and tetracycline antibiotics; and (3) the remedy for sarcoidosis according to (1) or (2), wherein the Propionibacterium acnes-targeting antibiotic is minocycline hydrochloride, clindamycin, ampicillin, or clarithromycin.

The present invention further relates to: (4) a method for treating sarcoidosis wherein a Propionibacterium acnes-targeting antibiotic is administered to a sarcoidosis patient; (5) the method for treating sarcoidosis according to (4), wherein the Propionibacterium acnes-targeting antibiotic is one or more antibiotics selected from penicillin antibiotics, cephalosporin antibiotics, macrolide antibiotics, lincomycin antibiotics, and tetracycline antibiotics; and (6) the method for treating sarcoidosis according to (4) or (5), wherein the Propionibacterium acnes-targeting antibiotic is minocycline hydrochloride, clindamycin, ampicillin, or clarithromycin.

### Brief Description of Drawings

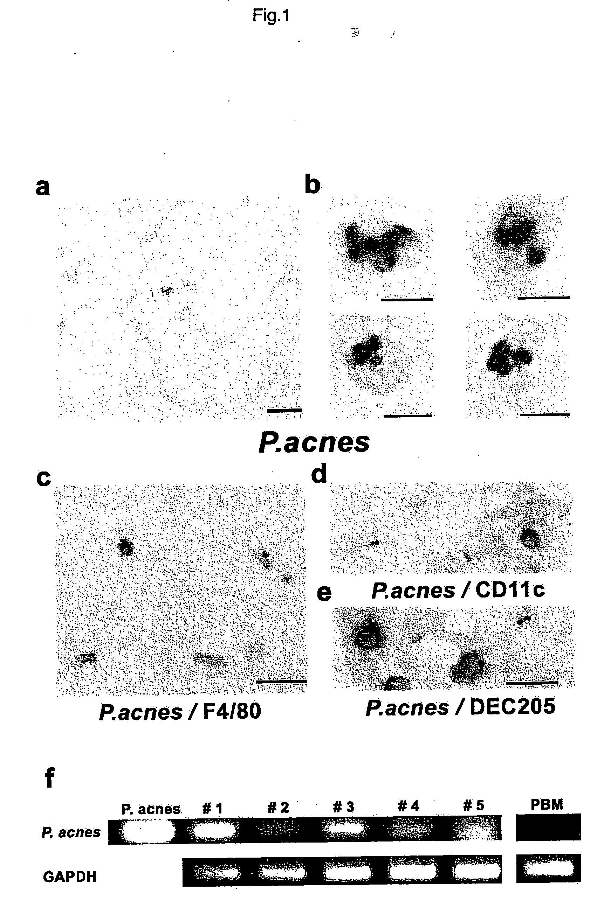

Fig. 1 is a set of photographs showing the experimental results indicating the existence of P. acnes in the alveolar of the healthy mouse lung.
   a,b: Immunostaining of P. acnes in the alveolar of the normal mouse lung (brown). High magnification of P. acnes-bearing cells . Scale bar; 5 µm (a), 20 µm (b).
   c to e: Double staining of P. acnes (brown) and F4 / 80 (blue) (c), double staining of P. acnes (brown) and CD11c (blue) (d), double staining of P. acnes (brown) and DEC205 (blue) (e). Only F4/80-presenting cells phagocytized P. acnes. Scale bar; 20 µm.
   f : Detection of P. acnes in the lower airway of the lung of healthy mice. Total RNAs extracted from live P. acnes were used as a positive control, and normal peripheral mononuclear blood cells were used as a negative control. Data shown are taken from representatives of three or more independent experiments. n = 5. Mice were numbered # 1 to 5.

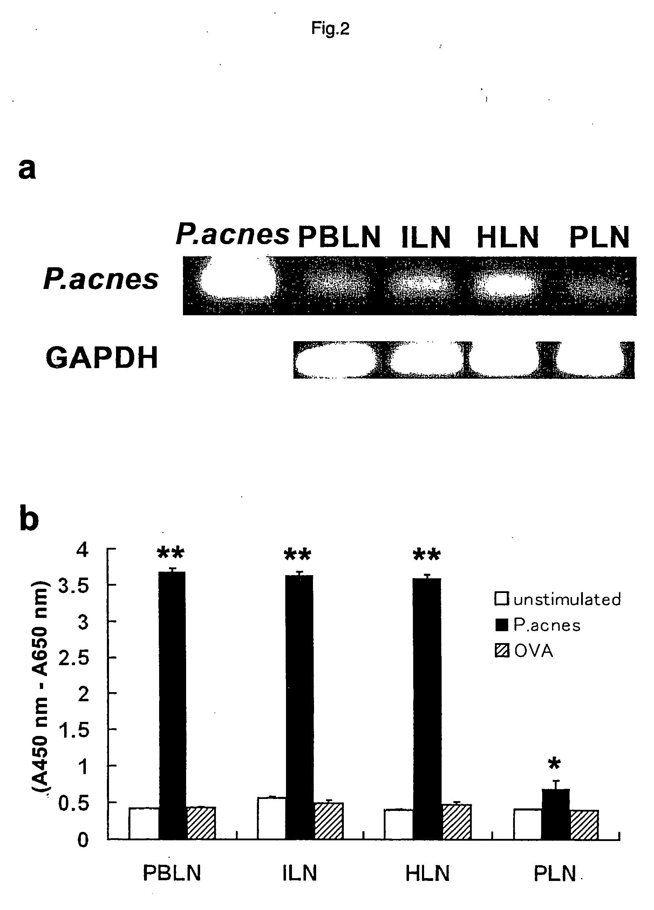

Fig. 2 is a set of photographs showing the results of immune response to P. acnes in the normal peripheral LNs.
   a: Detection of 16s ribosomal RNA of P. acnes in the normal peripheral LNs. Total RNAs extracted from live P. acnes were used as a positive control. Data shown are taken from representatives of three or more independent experiments.
   b: Proliferation assay of leukocytes responsive to P. acnes and the control antigen. White bar, unstimulated; black bar, P. acnes-stimulated; striped bar, OVA-stimulated. Data shown are taken from representatives of three or more independent experiments. n = 7. Data are means ± s.e.m. *, P < 0.05; **, P < 0.01, versus both unstimulated and OVA-stimulated groups.
Fig. 3 is a set of photographs showing the results of the adoptive transfer of P. acnes-sensitized helper T cells.
   a, b: H & E staining shows pulmonary (a) and hepatic (b) granulomas in mice injected with P. acnes-sensitized CD4⁺ cells on day 14.
   c, d: The lung (c) and the liver (d) of mice injected with unsensitized CD4⁺ cells corresponding to a and b are shown, respectively. Scale bar; 100 µm.
Fig. 4 is a set of photographs showing the results of repeated P. acnes immunization.
   a: H & E staining showed a number of pulmonary granulomas in the lungs of mice immunized three times, mainly in peripheral (upper panel) and peribronchovascular (lower panel) areas. Scale bar; 100 µm. B, bronchus; L, lymphatics; V, pulmonary vessels.
   b, c: Cellular components of pulmonary granulomas. CD4⁺ T cells (brown) at periphery of the granuloma, F4/80⁺ (b) and CD11c⁺ (c) cells (both blue) at the center of the granuloma. Scale bar; 100 µm. d: Th1/2 cytokine expression in the pulmonary granuloma. Granuloma CD4⁺ cells (green) expressed IFN-γ, but not IL-4 (both red). CD4⁺ IFN-γ⁺ cells (yellow) were distributed in the periphery of the layer of CD4⁺ cells. e to i: The number of BAL and its cellular components, serum calcium level, and ACE activity. The total number of BAL cells (e) and the number of lymphocytes in BAL (f) increased with the frequency of immunization, whereas the CD4/8 ratios (g) and serum calcium level (h) were maximum in the group immunized twice, and the serum ACE activity (i) of the group that received two or more immunizations increased. n = 5. Data are means ± s.e.m. (excluding h). Data shown are taken from representatives of three or more independent experiments. j, k: A high frequency of immunization induced a large number of hepatic granulomas (j) and aberrant accumulation of CD4⁺ T cells (arrows) in the red pulp of the spleen (k) . The samples were obtained from mice immunized nine times. Scale bar; 100 and 50 µm, respectively. RP, red pulp; WP, white pulp.

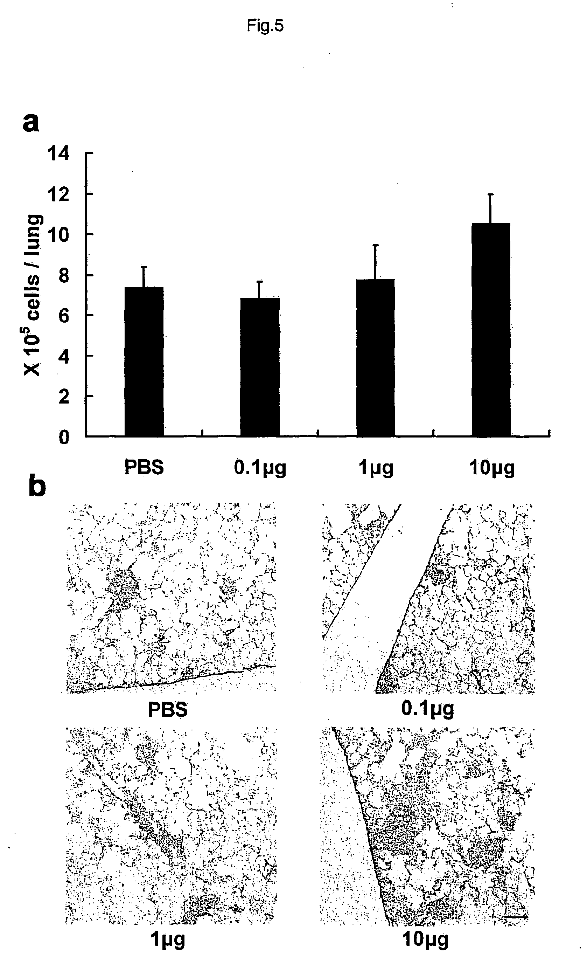

Fig. 5 is a set of photographs showing the influence of the amount of indigenous P. acnes colonies on granuloma formation.
   a: The total number of BAL cells from mice immunized three times with P. acnes, n = 5. Data are means ± s.e.m. Data shown are taken from representatives of three or more independent experiments.
   b: Histological findings by H & E staining. Scale bar; 100 µm.

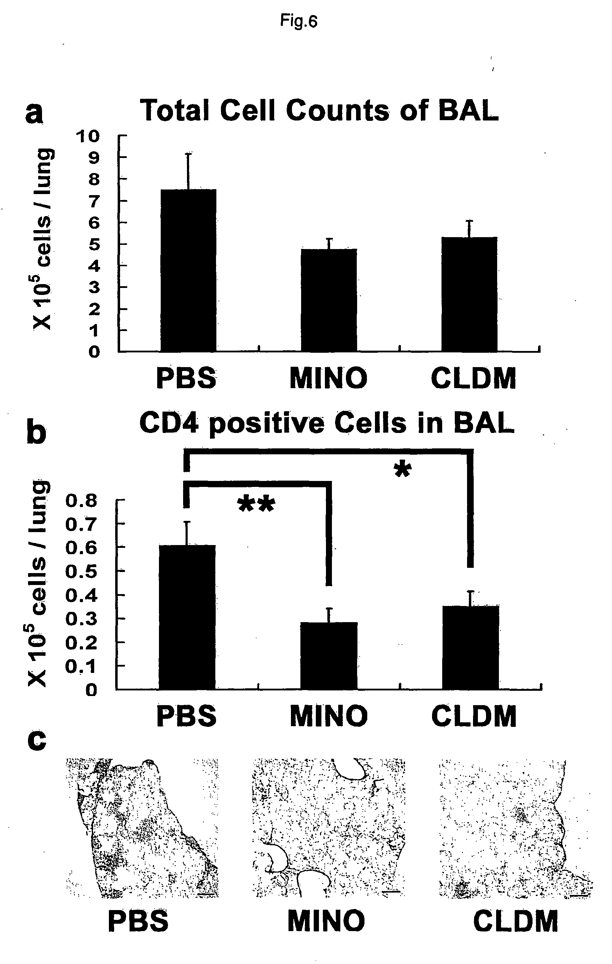

Fig. 6 is a set of photographs showing the therapeutic effect on sarcoidosis, achieved by administering the antibiotics of the present invention.
   a, b: The total number of BAL cells (a), CD4⁺ T cells (b) obtained from mice immunized three times with P. acnes. n = 4 to 6. Data are means ± s.e.m. *, P < 0.05; **, P < 0.01 (versus each PBS-treated group). Data shown are taken from representatives of three or more independent experiments.
   c: Histological findings by H & E staining. Scale bar; 100 µm.

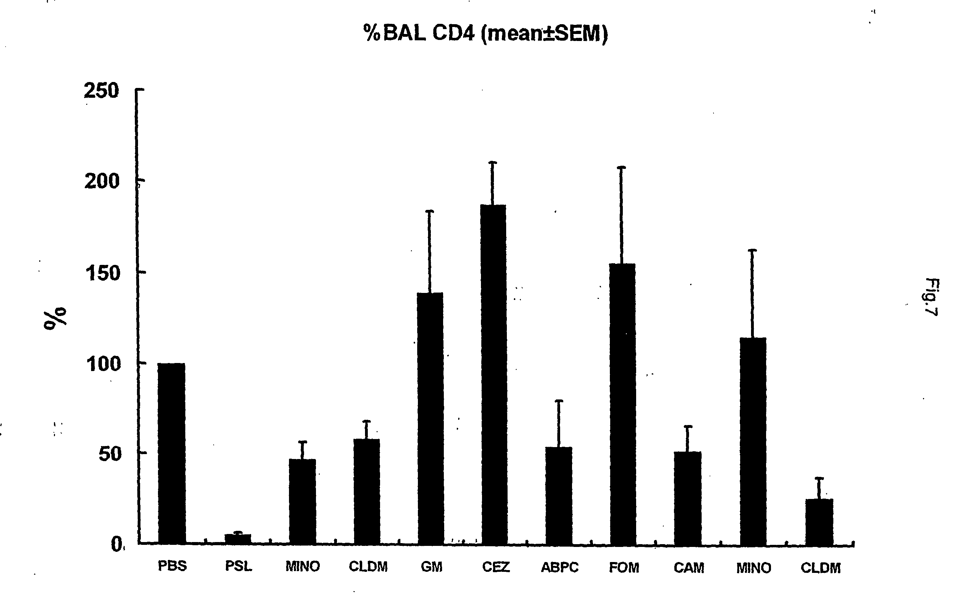

Fig. 7 is a view showing the results of examination of mice to be extrapulmonary immunized three times with P. acnes, for CD4⁺ cell ratios in BAL (bronchoalveolar lavage) versus penicillin: ampicillin (ABPC), cephem: cefazolin sodium (CEZ), aminoglycoside: gentamicin sulfate (GM), fosfomycin: fosfomycin (FOM), macrolide: clarithromycin (CAM). Further, this is a view showing the results of examination of groups administered for a short period as MINO short, CLDM short (administration of antibiotics was initiated one month after the treatment of granuloma-induced mouse model (when the last immunization was conducted)), for the number of CD4⁺ cells in BAL.

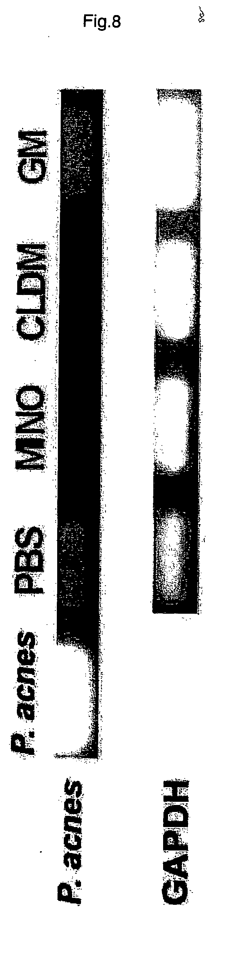

Fig. 8 is a set of photographs showing PCR data indicating that indigenous P. acnes in the lung was decreased by administration of antibiotics.

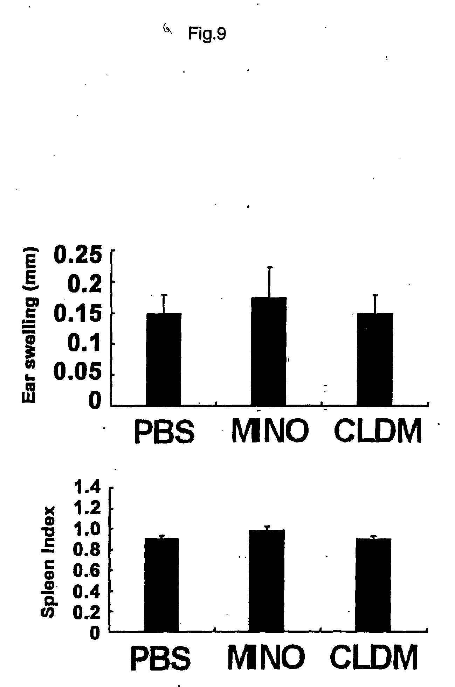

Fig. 9 is a set of views showing the results of examination whether nonspecific immunosuppressive phenomena are generated by administration of antibiotics.

### Best Mode of Carrying Out the Invention

As for the remedy for sarcoidosis of the present invention, there is no particular limitation as long as it is a remedy containingaP. acnes-targeting antibiotic as an active component. In addition, as for the method for treating sarcoidosis of the present invention, there is no particular limitation as long as it is a method for treating wherein a P. acnes-targeting antibiotic is administered to sarcoidosis patients. Here, sarcoidosis refers granulomatous diseases that extend to multiple organs, which are also called sarcoid, Boeck's sarcoid, Besnier-Boeck-Schaumann syndrome, angiolupoid, etc.

As the P. acnes-targeting antibiotic mentioned above, any substance can be used as long as it is a chemical substance having antibacterial activity to P. acnes, and examples include: penicillin antibiotics such as amoxicillin (AMPC), amoxicillin/clavulanate (AMPC/CVA), aspoxicillin (ASPC), benzylpenicillin (PCG), ampicillin (ABPC), bacampicillin (BAPC), ciclacillin (ACPC), piperacillin (PIPC); cephem antibiotics such as cefditoren pivoxil (CDTR-PI), cefetamet pivoxil hydrochloride (CEMT-PI), cefdinir (CFDN), cefixime (CFIX), cefcapene pivoxil (CFPN-PI), cefpodoxime proxetil (CPDX-PR); α-lactam antibiotics such as f aropenem sodium (FRPM), imipenem/cilastatin (IPM/CS), meropenem (MEPM), panipenem/betamipron (PAPM/BP) ; cephalosporin antibiotics such as ceftazidime (CAZ), cefalotin (CET), cefazolin (CEZ), cefotiam (CTM), cefotaxime (CTX), cefoperazone (CPZ), ceftizoxime (CZX), cefmenoxime (CMX), cefpirome (CPR), cefepime (CFPM), cefozopran (CZOP); macrolide/lincomycin antibiotics such as clindamycin (CLDM), lincomycin (LCM), erythromycin (EM), clarithromycin (CAM), rokitamycin (RKM); tetracyclines antibiotics such as minocycline (MINO), doxycycline (DOXY); quinolone, chloramphenicol (CP); rifamycin (RFM); sulfonamide (SA) drugs, cotrimoxazole; oxazolidinone; antibacterial antibiotics such as roxithromycin (RXM), vancomycin (VCM); synthetic antibacterial agents such as sparfloxacin (SPFX), ciprofloxacin (CPFX), levofloxacin (LVFX), tosufloxacin (TFLX), fleroxacin (FLRX). Among them, clindamycin, minocycline (minocycline hydrochloride), ampicillin, clarithromycin are preferable.

The remedy for sarcoidosis of the present invention can be also used as a preventive for sarcoidosis. In case where P. acnes-targeting antibiotic, an active component, is used as a medical remedy, various formulation components for drug preparation that are pharmaceutically acceptable and commonly-used, such as carriers, binders, stabilizers, fillers, diluents, pH buffers, disintegrators, solubilizers, auxiliary solubilizers, tonicity agents, etc., can be added. These remedies can be administered orally or parenterally. For example, they can be administered orally in the dosage form such as powders, granules, tablets, capsules, syrups, suspensions, etc., or they can be administered parenterally by injection in the dosage form such as solutions, emulsions, suspensions, etc. In addition, intranasal or transairway administration of the remedies is also possible in the form of sprays.

In case of formulations for oral administration, conventionally used various organic or inorganic carrier substances are used as pharmaceutically acceptable carriers. For example, fillers such as lactose and starch; lubricants such as talc and magnesium stearate; binders such as hydroxypropyl cellulose, polyvinyl pyrrolidone; disintegrators such as carboxymethyl cellulose, can be blended into tablets. Into formulations in a form of suspension, solvents such as saline alcohol; auxiliary solubilizers such as polyethylene glycol, propylene glycol; suspending agents such as stearyl triethanolamine, sodium lauryl sulfate, lecithin; tonicity agents such as glycerol, D-mannitol; buffers such as phosphate, acetate, citrate, can be blended. Further, if necessary, additives for formulation such as antiseptics, antioxidants, colorants, sweeteners can be also blended. In case of formulations for parenteral administration, water-soluble solvents such as distilled water,saline;auxiliarysolubilizers such as sodium salicylate; tonicity agents such as sodium chloride, glycerol, D-mannitol; stabilizers such as human serum albumin: preservatives such as methylparaben; local anesthetics such as benzyl alcohol, can be blended.

Further, the dose of the remedy for sarcoidosis of the present invention can be conveniently determined based on the types of diseases, age and body weight of patients, its administration forms, symptoms, etc. When administering to an adult, for instance, about 0.001 to 500 mg, preferably 1 to 50 mg of a P. acnes-targeting antibiotic and a pharmaceutically acceptable salt thereof as an active component are administered as a normal dose for one treatment, and it is desirable to administer this dose one to three times a day. As parenteral administration routes of the remedy for sarcoidosis of the present invention include, for example, intravenous, subcutaneous, intramuscular, intraspinal, transmucosal, and transairway administrations. Among them, intravenous, subcutaneous and transairway administrations are preferable.

### (Example 1)

The present invention is described belowmore specifically with reference to Examples, however, the technical scope of the present invention is not limited to these exemplification.

### (Results)

### [Existence of P. acnes in the alveoli of the healthy mouse lung]

When there is a preexisting immune response to P. acnes, it must be possible to detect P. acnes in the healthy lung. Therefore, the present inventors performed immunohistochemical analysis to examine the existence of P. acnes on fresh frozen lung sections collected from healthy C57BL/6 mice. Images of P. acnes-positive staining, wherein two to five round granules assembled, were observed. All of them were phagocytized by lung cells, most of which were adjacent to alveoli (Figs. 1a, b). Double immunostaining revealed that P. acnes-positive cells express a known macropharge marker, F4/80 (Fig. 1c), but not markers for dentritic cells such as CDllc (Fig. 1d) or DEC205 (Fig. 1e) (Blood. 95, 138-146, 2000, J. Immunol. 166, 2071-2079, 2001). In addition, the existence of P. acnes-genomes (Fig. If) was revealed by RT-PCR analysis of the normal lungs whose upper airways were removed, supporting the results of the immunostaining.

### [P. acnes-specific immune response of lymphocytes in a regional lymph node in the steady state]

For the purpose of presentation, peripheral APCs transport antigens to regional lymph nodes even in the steady state (Nature. 392, 245-252, 1998; J. Immunol. 167, 6756-6764, 2001), and P. acnes exists indigenously on the skin or the mucosal surfaces of the oral cavity and the intestine (Manual of Clinical Microbiology. 587-602, 1995) . For the test of P. acnes-specific immune response in the normal pulmonary lymph node, the present inventors demonstrated by RT-PCR that P. acnes genomes exist in the normal pulmonary lymph node as well as other lymph nodes (Fig. 2a). Subsequently, the present inventors tested whether P. acnes-specific immune response is established in these LNs, and found that lymphocytes in a regional lymph node of the lung specifically proliferate in response to P. acnes, as in the case of lymph node cells in the groin, the liver and the pancreas (Fig. 2b).

### [Pulmonary and hepatic granulomas were induced by intravenous adoptive transfer of P. acnes-sensitized T cells into untreated mice]

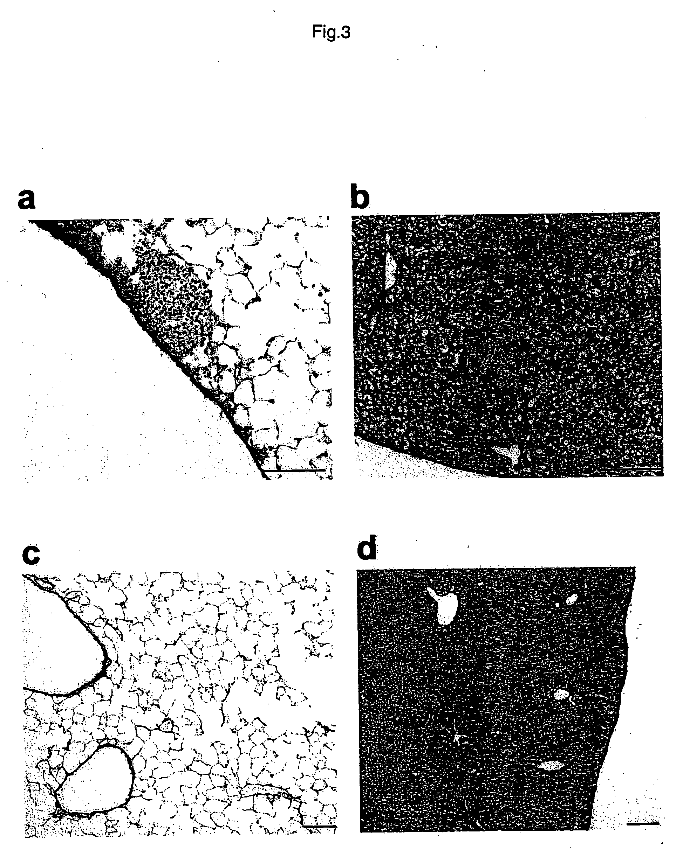

The present inventors next examined whether intravenous injection of P. acnes-sensitized T cells induces granuloma formation also in the normal lung and the normal liver. The present inventors obtained P. acnes-sensitized CD4⁺ T cells from a regional lymph node of a footpad, which had been repeatedly immunized with P. acnes, and the cells were injected into the tail vein of normal mice. Two weeks after the transplantation of 2 × 10⁶ T cells, the present inventors observed the changes in granulomas as aggregations of epithelioid and mononuclear cells in the lung and the liver (Figs. 3a, b). On the other hand, the adoptive transfer of unsensitized T cells did not indicate such results in the control experiment (Figs. 3c, d).

### [Pulmonary granulomas mimicking pulmonary sarcoidosis were induced by repeated immunization with P. acnes]

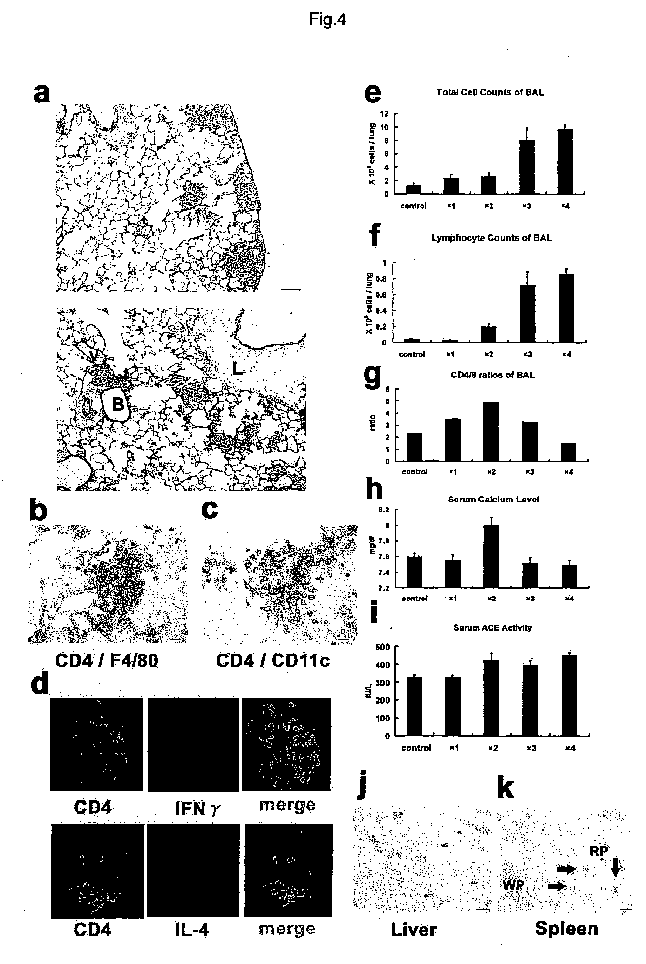

As an applied model of the above-mentioned transplant model, the present inventors continuously induced the supply, via circulation, of P. acnes-sensitized T cells to the normal mouse lung by repeated immunization via footpad. Characteristic granulomas were mainly formed in the subpleural and peribronchovascular regions in the lung of mice thus treated (Fig. 4a). It was revealed by the immunohistochemical analysis that the granulomas were constituted of antigen presenting cells at the center and CD4⁺ T cells at the periphery (Am. J. Respir. Cell Mol. Biol. 26, 671-679, 2002) (Figs. 4b, c). In addition, since these CD4⁺ T cells expressed not IL-4 but IFN-γ, it was suggested that the granulomas were type Thl (Fig. 4d). The present inventors could predict the level of granulomatous lesions by counting the number of BAL cells. The total number of total leukocytes and lymphocytes in BAL fluid increased in a manner dependent on the frequency of administration (Figs. 4e, f), however, the maximum CD4/8 cell ratio was observed in the group injected twice (Fig. 4g).

As the results obtained from this experimental model were consistent with the characteristics of sarcoidosis patients, the present inventors examined the serological similarities by evaluating serum calcium levels and ACE (angiotensin-converting enzyme) activities (N. Engl. J. Med. 336, 1224-1234, 1997; Lancet. 361, 1111-1118, 2003; Diagnosis of Disease of the CHEST Vol. 1, 1533-1583, 1999). Serum calcium increased most largely in the group injected twice (Fig. 4h), and ACE activities increased in an antigen dose-dependent manner (Fig. 4i). By immunohistochemical analysis of the liver and the spleen in which lesions are frequently observed in sarcoidosis, numerous granulomas in the liver (Fig. 4j) and aberrant accumulation of CD4⁺ T cells in the red pulp of the spleen were observed in the frequently immunized mice.

### [Increase of indigenous P. acnes in the healthy lung enhanced the pulmonary granuloma formation]

Provided that P. acnes that exists indigenously in the healthy lung causes pulmonary granuloma, the amount of such P. acnes should affect on the level of lesions. In order to verify this hypothesis, the present inventors preadministered live P. acnes to healthy mouse lungs before immunization. To exclude the possibility that intratracheal administration alone induces granulomas, the present inventors confirmed that there were no glanulomas in the control lungs at either the initial stage or the final stage of the experiment. The total number of leukocytes in BAL collected after three immunizations increased in a manner dependent on the number of P. acnes preadministered (Fig. 5a), and the results of histological examination of granulomatous lesions were consistent with this observation (Fig. 5b).

### [Antibiotic treatment alleviated granulomatous lesions in mouse sarcoidosis lung]

For further evaluation of the importance of indigenously existing P. acnes, the present inventors decreased the number of indigenously existing P. acnes in the healthy lungs with the use of antibacterial substances, minocycline hydrochloride (MINO) and clindamycin (CLDM), before immunization (J. Eur. Acad. Dermatol. Venereol. 15, 51-55, 2001; Semin. Cutan. Med. Surg. 20, 139-143, 2001). Two weeks after the third immunization, the MINO- and CLDM-treated mice exhibited marked decrease in the total number of BAL leukocytes (Fig. 6a); the number of CD4⁺ BAL cells in these 2 groups decreased by 53.5% and 42.1%, respectively (Fig. 6b). By histological examination, decrease of granulomatous lesions was also revealed (Fig. 6c).

In addition, CD4⁺ ratios in BAL (bronchoalveolar lavage) versus penicillin: ampicillin (ABCP), cephem: cefazolin sodium (CEZ), aminoglycoside: gentamicin sulfate (GM), fosfomycin: fosfomycin (FOM), macrolide: clarithromycin (CAM) were examined as in the case of the above-mentioned minocycline hydrochloride and clindamycin. Further, the examination was conducted also for a group administered for a short period as MINO short, CLDM short (administration of antibiotics was initiated one month after the treatment of granuloma-induced mouse model (when the last immunization was conducted)). The results are shown in Fig. 7. The values in Fig. 7 are indicated as percentage in comparison to the control (PBS) group whose values are set at 100. As a result, marked decrease of the total number of BAL leukocytes was observed in the groups administered with ABPC (improved by 46.1%), CAM (improved by 48.3%), CLDM short (improved by 74.3%), in addition to the groups administered with MINO (improved by 53.5%), CLDM (improved by 42.1%) mentioned above. Though no effect was observed in GM, this is consistent with the fact that GM is originally hyporesponsive to P. acnes.

### [Administration of antibiotics decreased indigenous P. acnes genomes in the lung]

Whether indigenous P. acnes in the mouse lung is decreased by antibiotics was examined by PCR using PBS as a control, and the results are shown in Fig. 8. The results indicated that P. acnes decreased when using MINO and CLDM. In the case where GM was used, P. acnes did not decrease so much as in the case where MINO or CLDM was used. This is consistent with the fact that GM is originally hyporesponsive to P. acnes, and is in concert with the results of BAL shown in Fig. 7.

### [Granunolmaous lesions were improved by antibacterial effect of antibiotics]

Whether MINO and MONO, which have an antibiotic function to sarcoidosis, cause a nonspecific immunosuppression phenomenon was examined. Whether MINO and CLDM improve the size of ear swelling or spleen index was examined, and the results are shown in Fig. 9. As a result, no marked differences were observed in comparison to PBS, which is a control. This fact revealed that granunolmaous lesions were improved by true antibacterial effect of antibiotics.

### (Example 2)

### (Discussion)

Though living organisms are constantly exposed to foreign antigens, it has been considered that the lower airway of the lung is an inviolable germ-free space, and that entry of pathogens into the lung causes pulmonary disorders. Based on this assumption, animal models for pulmonary disorders were constructed by forced administration of antigens via the trachea, nasal cavity, or antigen-embolized pulmonary vessels (Am. J. Pathol. 158, 1503-1515, 2001; J. Immunol. 166, 3423-3439, 2001; Nature. 392, 245-252, 1998; Immunology. 108, 352-364, 2003). However, clinicians are often confronted with cryptogenic pulmonary disorders without evident exposure to pathogens, in particular, interstitial pulmonary disorders. Accordingly, the present inventors hypothesized that there may be an indigenous organism in the healthy lung that can act as a pathogen under certain conditions.

P. acnes distributes on the skin and mucosal surface of healthy individuals, acts as a pathogen of acne vulgaris (Semin. Cutan. Med. Surg. 20, 139-143, 2001), and remarkably induces granuloma formation in experimental models (J. Exp. Med. 193, 35-49, 2001; J. Exp. Med. 195, 1257-1266, 2002), and therefore, P. acnes is considered to be a strong candidate as a pathogen. In fact, some previous reports emphasized a correlation between P. acnes and sarcoidosis (Lancet. 354, 120-123, 1999; J. Clin. Microbiol. 40, 198-204, 2002). As mentioned above, the present inventors indentified P. acnes in normal mouse alveolar cells by immunostaining (Figs. 1a, b). These P. acnes-bearing cells expressed F4/80, but not CD11c or DEC205, and this is consistent with known finding about macrophages to phagocytize antigens and deliver antigen information to dentritic cells in the lung (Figs. 1c to e) (Am. J. Respir. Crit. Care Med. 162, S151-S156, 2000; Immunology. 81, 343-351, 1994). After examining the existence of antigen presenting cells (APCs) that phagocytize P. acnes in the healthy lung, the present inventors examined the existence of immune response to P. acnes in regional lymph nodes of the normal mouse lung. Indeed, lymphocytes in the normal pulmonary lymph nodes exhibited P. acnes-specific proliferation (Fig. 2b), suggesting that these cells already established immune response to P. acnes in the steady state by APC derived from the lung.

The present inventors subsequently hypothesized that P. acnes-sensitized T lymphocytes cause pulmonary inflammation even without artificial antigen-anchoring. Adoptive transfer of P. acnes-sensitized CD4⁺ T cells to untreated mice caused granulomatous changes in the lung and the liver (Fig. 3a). This indicates that P. acnes-sensitized CD4⁺ T cells in extrapulmonary lymph nodes can induce granuloma formation by entry into the normal lung via circulation. Therefore, the present inventors hypothesized that continuous extrapulmonary sensitization of normal mice with P. acnes results in continuous supply of P. acnes-sensitized T cells, and leads to chronic pulmonary granuloma formation. These mice exhibited distinct pulmonary granulomas in lymph-rich regions such as the subpleural, pleura 1, and perivascular regions (Scientific Foundations, Vol. 1, 2395-2409, 1997) (Fig. 4a), and showed typical granulomas (Scientific Foundations, Vol. 2, 2395-2409, 1997) (Figs. 4b, c) and the expression of Th1 cytokines (Fig. 4d). These features closely resemble those of pulmonary sarcoidosis (N. Engl. J. Med. 336, 1224-1234, 1997; Curr. Opin. Pulm. Med. 8, 435-440, 2002; Diagnosis of Disease of the CHEST Vol. 1, 1533-1583, 1999) . Further, mouse models exhibited increased ACE activity (Fig. 4i) and enhanced calcium level (Fig. 4h) as well as increased ratios of CD/CD8 cells in BAL (Fig. 4g). These observations are consistent with those of previous studies demonstrating a positive correlation between serum calcium levels and the BAL CD4/CD8 ratios in sarcoidosis patients (Am. J. Med. 110, 687-693, 2001). In addition, the present inventors found similar extrapulmonary lesions in this mouse model, in the liver and the spleen, which are frequently affected in sarcoidosis (Figs. 4j, k) (N. Engl. J. Med. 336, 1224-1234, 1997; Lancet. 361, 1111-1118, 2003; Diagnosis of Disease of the CHEST Vol. 1, 1533-1583, 1999). The above observations indicated that the model repeatedly immunized with P. acnes exhibited remarkable similarity to sarcoidosis patients.

As the entry of P. acnes-sensitized T cells into the lung via circulation may induce granuloma formation (Fig. 3a), the interaction between the lung APCs phagocytizing P. acnes and T cells in the lung lymph node was considered to be essential to granulama formation. To confirm this, whether changes in the total number of indigenous P. acnes in the healthy lung have an effect on pulmonary granuloma formation was examined. As expected, decrease of P. acnes by treatment with antibacterial substances decreased pulmonary granulomatous lesions, whereas intrapulmonary preadministration of P. acnes aggravated pulmonary granulomatous lesions (Figs. 6a, b). These results suggest not only that indigenous P. acnes plays an extremely important role in pulmonary granuloma formation by extrapulmonary P. acnes sensitization, but also clinically useful for sterilizing treatments with antibacterial substances as a pulmonary sarcoidosis treatment.

The etiology of sarcoidosis remains to be elucidated. Immunosuppuressive treatment mainly with corticosteroid have been employed for this disease for more than 50 years, however, the long-term effect of steroidal treatment on chronic pulmonary sarcoidosis is still under discussion (Lancet. 361, 1111-1118, 2003), and its high relapse rate after treatment often becomes a clinical problem (Chest. 111, 623-631, 1997). InthisExample, novel mouse pulmonary granuloma model closely resembling pulmonary sarcoidosis was constructed. As suggested by the present inventors, if P. acnes exists in the lung of healthy person, there is a possibility that pulmonary lesions may occur subsequent to excessive sensitization with P. acnes in extrapulmonary areas such as acne vulgaris in persons with unique genetic background as reported in sarcoidosis patients (N. Engl. J. Med. 336, 1224-1234, 1997; Lancet. 361, 1111-1118, 2003; J. Immunol. 167, 6756-6764, 2001) . Therefore, the eradication of this pathogen should be considered prior to the conventional immunosuppressive treatment of sarcoidosis. The present inventors suggest that this novel concept for pulmonary sarcoidosis is worth studying further, and provides the basis for new therapeutic strategy.

### (Example 3)

### (Materials and methods)

### [Mice]

Female C57BL/6J mice of 5 to 7 weeks of age were obtained from CLEA Japan (Shizuoka, Japan) or Japan SLC, Inc. (Tokyo, Japan), and kept under specific pathogen-free (SPF) conditions in the animal facility of the Department of Molecular Preventive Medicine, Graduated School of Medicine, the University of Tokyo. All animal experiments were conducted in accordance with the guidelines of the University of Tokyo.

### [Immunostaining]

The following anti-mouse monoclonal antibodies (mAbs) were used. CD4 (clone; RM4-5), biotinylated IFN-γ (XMG1.2), biotinylatedIL-4 (BVD6-24G2), all fromBD Pharmingen San Diego, California); biotinylated F4/80 (CI:A3-1), CDllc (N418), both from Serotec (Oxford, UK); DEC-205 (NLDC-145; BMA Biomedical, Augst, Switzerland); and mouse mAb to P. acnes recognizing lipoteichoic acid of the plasma membrane (J. Exp.Med. 193, 35-49, 2001).

As secondary antibodies, alkaline phosphatase-labeled anti-rat IgG antibody (Jackson ImmunoResearch Laboratories, West Grove, Pennsylvania), alkaline phosphatase-labeled anti-hamster IgG antibody (Cederlane, Ontario, Canada), or avidin (Nichirei Corporation, Tokyo, Japan), and peroxidase-labeled anti-rat Ig antibody (BioSource, Camarillo, California), or peroxidase-labeled anti-mouse Ig antibody (DAKO, Carpinteria, California) were used.

Single and double immunostaining were conducted by the indirect immunoalkaline phosphatase and immunoperoxidase methods (J. Exp. Med. 183, 1865-1878, 1996). For double immunostaining, acetone-fixed 6-µm fresh frozen tissue sections were incubated with anti-CD4 antibodies and then Alexa Fluor 488 anti-rat Ig antibodies (Molecular Probes, Eugene, Oregon). Next, they were incubated with biotinylated IFN-γ or biotinylated IL-4, and further incubated with Alexa 594-conjugated avidin (Molecular Probes), and observed by fluorescence microscopy (Clin. Immunol. 97, 33-42, 2000).

### [RT-PCR]

Samples of 1 µg of total RNA were isolated from the lungs, and regional lymph nodes samples of the lung, the liver, the skin and the pancreas of SPF mice using Trizol (Invitrogen, Groningen, the Netherlands) according to the manufacturer's instructions. Then, RNA samples were reverse transcribed into cDNA, and amplified (J. Exp. Med. 193, 35-49, 2001; J. Clin. Invest. 102, 1933-1941, 1998). PCR products of 16s ribosomal RNA of P. acnes were electrophoresed on 2.5% agarose gel. The bands visualized by ethidium bromide staining were expected size for each mRNA product. Oligonucleotide primers for P. acnes were designed as described previously (J. Clin. Microbiol. 40, 198-204, 2002): forward, 5'-GCGTGAGTGACGGTAATGGGTA-3' (SEQ ID NO: 1); reverse, 5'-TTCCGACGCGATCAACCA-3' (SEQ ID NO: 2). Contamination of P. acnes during the experiment was checked by buffer control. As primers for GAPDH as an internal standard, previously described ones were used (J. Exp. Med. 193, 35-49, 2001). PCR conditions: heated at 95°C for 5 min, followed by 40 cycles of 95°C for 30 sec, 58°C for 60 sec, 72°C for 60 sec, and finally heated at 72°C for 10 min.

### [Antigen-specific proliferation assay]

In vitro cell proliferation assay was conducted according to the method described previously (J. Exp. Med. 193, 35-49, 2001). In brief, peribronchial, axillary, groin, hepatic, and pancreatic lymph node cells (10⁵ cells/190 µl/well) from normal mice were stimulated with antigens (P. acnes and OVA; 10 µg/10 µl of culture medium) at 37°C for 72 hours. After incubation, cell proliferation was measured with Premix WST-1 cell proliferation measuring system (Takara Bio Inc. , Shiga, Japan) according to manufacturer's instructions.

### [Adoptive transfer of P. acnes-sensitized helper T cells]

P. acnes-sensitized CD4⁺ T cells were isolated from groins of normal mice and mice immunized three times. Immunization was conducted by subcutaneous injection of 400 pg of heat-killed P. acnes (ATCC11828, American Type Culture Collection, Manassas, Virginia) and Freund's complete adjuvant (Difco, Detroit, Michigan) into the footpad at 2-week intervals. CD4⁺ cells were isolated with the use of MACS system (Miltenyi Biotech, Bergisch Gladbach, Germany) according to manufacturer's instructions. The purity of CD4⁺ cell populations was 94% or higher, as confirmed by immunostaining flow cytometry. The isolated CD4⁺ cells (2 × 10⁶ cells/PBS 200 µl) were injected into the tail vein of normal mice, and histological analysis of the lungs was conducted two weeks after the injection.

### [Flow cytometric analysis of bronchoalveolar lavage (BAL) cells

BAL cells were collected by five injections of 0.8 ml of sterile PBS containing 2% FCS (Sigma, St. Louis, Missouri) and 2 mM EDTA. The total number of BAL leukocytes was counted with a hemocytometer. Before the analysis with EPICS Elite instrument (Beckman Coulter, Miami, Florida), BAL cells were preincubated with rat anti-mouse CD16/CD32 (clone; 2.4G2) mAb to block FcR-mediated binding, and then incubated for 25 min at 4°C with FITC-conjugated anti-CD4 (H129.19) mAb and PE-conjugated anti-CD8α (53-6.7) mAb, both from BD Pharmingen.

### [Serological analysis]

Serum calcium levels were measured with Fuji DRI-CHEM 5500V (Fuji Medical System, Tokyo, Japan) and angiotensin-converting enzyme (ACE) activity was measured with ACE color (Fuji Medical System, Tokyo, Japan) according to the manufacturer's instructions.

### [Antibiotic treatment]

Minocycline hydrochloride (MINO) (Wyeth Ledele, Tokyo, Japan) and clindamycin (CLDM) (Pharmacia, Tokyo, Japan) were used. On day one, 133 pg of MINO and 1.6 mg of CLDM were administered intratrancheally (i.t.). Subsequently, the same dose of each antibiotic was injected intraperitoneally (i.p.) everyday for one week before immunization, then intraperitoneal injection was conducted three times per week. During the experiment, mice were given water containing each antibiotic at the same dose as mentioned above. Similarly, ampicillin (ABPC), cefazolin sodium (CEZ), gentamicin sulfate (GM), fosfomycin (FOM), clarithromycin (CAM) were used and single dose of these antibiotics was set by calculating the maximum amount for regular use by adults based on their body weight.

### [Statistical analysis]

Differences were evaluated using the two factors, in other words, factorial analysis of variance (ANOVA) and Fisher's protected least significant difference. P. values < 0.05 were considered statistically significant.

### Industrial Applicability

The present invention makes it possible to provide a remedy for sarcoidosis, one of systemic granulomatous diseases, and a method for treating sarcoidosis.

## Claims

1. A remedy for sarcoidosis containing a Propionibacterium acnes-targeting antibiotic as an active component.

2. The remedy for sarcoidosis according to claim 1, wherein the Propionibacterium acnes-targeting antibiotic is one or more antibiotics selected from penicillin antibiotics, cephalosporin antibiotics, macrolide antibiotics, lincomycin antibiotics, and tetracycline antibiotics.

3. The remedy for sarcoidosis according to claim 1 or 2, wherein the Propionibacterium acnes-targeting antibiotic is minocycline hydrochloride, clindamycin, ampicillin, or clarithromycin.

4. A method for treating sarcoidosis wherein a Propionibacterium acnes-targeting antibiotic is administered to a sarcoidosis patient.

5. The method for treating sarcoidosis according to claim 4, wherein the Propionibacterium acnes-targeting antibiotic is one or more antibiotics selected from penicillin antibiotics, cephalosporin antibiotics, macrolide antibiotics, lincomycin antibiotics, and tetracycline antibiotics.

6. The method for treating sarcoidosis according to claim 4 or 5, wherein the Propionibacterium acnes-targeting antibiotic is minocycline hydrochloride, clindamycin, ampicillin, or clarithromycin.


SEQUENCE LISTING

<110> Japan Science and Technology Agency

<120> The curative medicine and the medical treatment method of sarcoidosis

<130> B20-01PCT

<150> JP2003-270809
<151> 2003-07-03

<160> 2

<170> PatentIn version 3.1

<210> 1
<211> 22
<212> DNA
<213> Artificial

<220>
<223> forward primer

<400> 1
gcgtgagtga cggtaatggg ta 22

<210> 2

<211> 18

<212> DNA

<213> Artificial

<220>

<223> reverse primer

<400> 2

ttccgacgcg atcaacca 18